# EUROPÄISCHE PATENTANMELDUNG

(11) **EP 2 455 678 A2**
(43) Veröffentlichungstag der Anmeldung: **23.05.2012**
(21) Anmeldenummer: 11009257.4
(22) Anmeldetag: 22.11.2011
(51) Int. Cl.: F24F 13/16

(54) **Vorrichtung und Verfahren zur Entkeimung strömender Luft**

(30) Priorität: 23.11.2010 DE 102010052053
(71) Anmelder: Schröder, Werner, 31542 Bad Nenndorf (DE)
(72) Erfinder: Schröder, Werner, 31542 Bad Nenndorf (DE)
(74) Vertreter: Lins, Martina

(57) **Zusammenfassung**

Die Erfindung bezieht sich auf ein Verfahren zur Entkeimung von Luft, bei welchem die potentiell mit pathogenen Keimen beladene Luft mit UV-Licht bestrahlt wird. Für besondere Aufgaben, insbesondere im klinischen Bereich, wird angestrebt, dass auch besonders gefährliche oder schwer abzutötende Keime (Viren und Bakterien) in einem Luftstrom vollständig unschädlich gemacht werden. Hierfür wird der Luftstrom innerhalb einer Behandlungskammer (1) eines Gerätes (10) quer zu Längsachse eines stabförmiges UVC-Strahlers (2) durch einen angrenzend an die Oberfläche des Strahlers ausgebildeten Spalt (3) hindurchgeführt, wobei der Luftstrom so zwangsgeführt wird, dass jedes Teilvolumen während des Durchtritts einer Strahlungsleistung von mindestens 30 mW/cm² ausgesetzt wird.

## Beschreibung

Die Erfindung betrifft ein Verfahren zur Entkeimung von Luft, bei welchem die potentiell mit pathogenen Keimen beladene Luft innerhalb eines Gerätes oder einer Bestrahlungseinheit in einem Luftstrom geführt und mit UVC-Licht bestrahl wird, und eine Vorrichtung zur Entkeimung strömender Luft mit UV-Strahlung, die besonders für die Durchführung des Verfahrens geeignet und ausgelegt ist.

An öffentlichen Orten, in Krankenhäusern und Einrichtungen, in denen zahlreiche Menschen zusammenkommen, aber auch im privaten Bereich kann die Luft mit Keimen wie Bakterien und Viren so belastet sein, so dass sie laufend entkeimt werden sollte.

Es ist bekannt, dass manche Bakterien und Viren mit UV-Strahlung abgetötet werden können. Über den Zusammenhang zwischen Strahlungsdauer oder - intensität und Wirksamkeit gibt es Untersuchungen. Es wird vermutet, dass die Wirkung der UV-Strahlung darauf beruht, dass das Erbgut der Mikroorganismen geschädigt wird, so dass sie sich nicht weiter replizieren können und absterben oder gänzlich zerstört werden.

Für jede technische Entkeimung ist es erforderlich, dass die keimtötende Wirkung reproduzierbar, d.h. zuverlässig ist und sich mit großer Sicherheit auf möglichst alle Typen von Bakterien und Viren erstreckt. Dies konnte bislang nicht gewährleistet werden.

Aus der US 599,761 A ist ein in sich geschlossenes (Umluft)-Luftreinigungssystem bekannt, bei dem Luft durch eine Standgerät mit Ein- und Ausgangsfiltern umgewälzt wird. Im Innern des Geräts befindet sich eine Behandlungskammer, die mit germiziden UV-Strahlern ausgestattet ist. Die Luft wird mit einem Ventilator in das Gerät und durch einen dreistufigen Primärfilter mit HEPA-Filteranteil eingesogen, innerhalb der Behandlungskammer in einem Wirbelstrom an den Strahlern vorbeigeführt und nach oben und außen durch einen Ausgangsfilter entlassen. Das Gerät kann ca. 600 m³ Luft pro Stunde behandeln. Die Intensität der Strahler soll so gewählt werden, dass mehr als 90 % der nicht im Primärfilter verbleibenden Teilchen zerstört werden.

Das bekannt Gerät hat verschiedene Nachteile. Der relativ teure HEPA-Spezialfilter wird vor der eigentlichen germiziden Behandlung den pathogenen Keimen ausgesetzt und verkeimt daher schnell. Obwohl die Verwirbelung des Luftstroms einen Schlupf an unbehandelten Luft-Teilströmen ausschließen soll, wird der Wirkungsgrad lediglich mit "oberhalb 90 %" angegeben. Für gefährliche Keime und in sensibler Umgebung ist dies jedoch oft nicht ausreichend.

Ein weiteres Umluft-Standgerät ist aus der US 5,616,172 A bekannt. Die Luft wird von unten in das turmartige Gerät eingezogen und nach oben abgeführt. In diesem Gerät ist der HEPA-Filter der UV-Behandlung nachgeschaltet. Die UV-Strahler sind lotrecht, also längs zur Strömungsrichtung angeordnet. Stromaufwärts von diesen befindet sich ein Vorfilter, der unter anderem verhindert, dass Feststoffpartikel in das Gerät gelangen, z.B. durch Rauch, die sich auf den Strahlern absetzen und deren Leistung vermindern können. Aufgrund der laminaren Strömung durch den UV-Behandlungsraum werden verschiedene Bereiche der Luftströmung unterschiedlich intensiv erfasst. Ein hierzu ähnliches Gerät ist auch in der US 4,210,429 offenbart.

Der Erfindung liegt die Aufgabe zugrunde, pathogene Keime, nämlich Viren und Bakterien, auf einfache und sichere Weise mit einem kompakten und wartungsarmen Gerät soweit aus Luft oder Gasen zu entfernen, dass keine messbare Restinfektiosität mehr vorhanden ist.

Diese Aufgabe wird durch das erfindungsgemäße Verfahren zur Entkeimung von Luft gelöst, bei welchem die potentiell mit pathogenen Keimen beladene Luft innerhalb eines Gerätes oder einer Bestrahlungseinheit in einem Luftstrom geführt und mit UVC-Licht bestrahlt wird und bei dem der Luftstrom quer zur Längsachse wenigstens eines stabförmigen UVC-Strahlers durch wenigstens einen, angrenzend an die Oberfläche des Strahlers ausgebildeten Spalt hindurchgeführt wird und so zwangsgeführt wird, dass jedes infinitesimale Teilvolumen einer Strahlungsleistung von mindestens 30 mW/cm² ausgesetzt wird.

Bevorzugt bewirkt die Zwangsführung, dass jedes Teilchen im Luftstrom die Oberfläche eines Strahlers in einem Abstand von kleiner gleich 4 cm passieren muss.

Die Erfindung liegt die Erkenntnis zugrunde, dass dem Aspekt der Strahlungstiefe bislang zu wenig Beachtung geschenkt wurde. Die Lichtintensität einer Strahlungsquelle nimmt mit dem Quadrat der Entfernung ab. In Versuchen wurde gefunden, dass Keime aller Art sicher abgetötet wurden, wenn sie mit Licht im UVC-Bereich, jedoch vorzugsweise nicht unterhalb von 200 nm, auch nur kurze Zeit bei hoher Intensität - das heißt unausweichlich nahe der Strahlungsquelle - bestrahlt werden. Es kommt also bei der Erfindung darauf an, kurzzeitig eine hohe Strahlungsintensität anzuwenden, die jeden einzelnen Keim erfasst. Dabei genügt es nicht, bzw. ist es nicht gleichwertig, in einem größeren mit Strahlern ausgerüsteten Raum über eine längere Zeit zu bestrahlen. Auch wenn in dem keimbelasteten Gas (meist Luft) Konvektion herrscht oder das zu behandelne Gas umgewälzt wird, können sich Keime dauerhaft an einem von der Strahlungsquelle entfernten Ort, z.B. nahe einer Wand des Raumes oder auch innerhalb einer Ansammlung mehrerer Keime aufhalten. Solche Keime, die nicht hinlänglich intensiv bestrahlt werden, können überleben. Bei strömenden Verhältnissen kann es dann einen "Schlupf" geben, der es einzelnen Keimen erlaubt, nur mit geringeren Strahlungsintensitäten in Berührung zu kommen. Deswegen kann in den einleitend beschriebenen Systemen keine ausreichende Entkeimung gewährleistet werden.

Unter UVC-Strahlung wird generell Strahlung bis ca. 300 nm und im engeren Sinne bis ca. 280 nm verstanden. Die Quecksilberbestrahlungslinie bei 253 nm besitzt bekanntermaßen eine stark keimtötende Wirkung, da die Einstrahlung in lebende Materie bei dieser Wellenlänge zu einer Dimerisierung bestimmter DNA-Bausteine und zum Absterben der Zellen führt. Sehr kurzwellige UVC-Strahlung sollte nicht eingesetzt werden, da sie aus dem Luftsauerstoff Ozon bildet, welches aus der Luft wieder entfernt werden müsste. Dieser Aufwand kann dadurch vermieden werden, dass die UVC-Bestrahlung praktisch ausschließlich mit Licht oberhalb 200 nm Wellenlänge - weiter bevorzugt mit Quecksilberlicht mit einem breiten Wellenlängenmaximum um etwa 250 bis 300 nm - erfolgt, indem Wellenlängen unterhalb von 200 nm ausgefiltert oder nicht abgestrahlt werden. Da Ozon selbst keimtötende Wirkung hat, ist es überraschend, dass bei der Erfindung mit einer kurzzeitigen, intensiven Bestrahlung auch ohne die Anwesenheit von Ozon eine so vollständige keimtötende Wirkung erzielt wird.

Das erfindungsgemäße Verfahren verknüpft auf diese Weise verschiedene Vorzüge. Durch die Zwangsführung und die in geringem Abstand von der Strahleroberfläche intensive Kurzzeitbestrahlung wird eine sehr gute keimabtötende Wirkung erzielt, die einer Behandlung bei geringerer Intensität über einen längeren Zeitraum überlegen ist. Gleichzeitig wird durch die Zwangsführung jeder Schlupf verhindert, was durch Verwirbelungseffekte beim Querumströmen des stabförmigen Strahlers unterstützt wird. Gemeinsam ergibt dies eine große Effektivität der Behandlung.

Bei Verwendung von Strahlung mit Wellenlängen oberhalb 200 nm ergibt sich der weitere Vorteil, dass Ozon unmittelbar vermieden wird, was das Verfahren unkompliziert und anwendungssicher macht, denn das Ozon muss nicht mehr nachträglich entfernt werden. Kosten, die für die nachträgliche Entfernung des Ozons notwendig wären, beispielsweise für ein Katalysator, können hierdurch entfallen. Dennoch ist die Behandlungsgesamtwirkung nicht vermindert, wenn auf das zusätzlich keimtötende Ozon verzichtet wird.

In bevorzugter Ausführungsform werden für das Verfahren UVC-Strahlungsquellen mit Wellenlängenausschluss unterhalb 200 nm verwendet (sogenannte nicht-ozonbildende UVC-Strahler, im Handel erhältlich).

Vorzugsweise wird der Wellenlängenausschluss unterhalb 200 nm mit Hilfe einer für die Strahlung teildurchlässigen Wand bewirkt. Die Wand kann eine den Strahler vorzugsweise in jede Abstrahlungsrichtung umgebende Außenwand des Strahlers oder eine zusätzlich außen um den Strahler angeordnete Wand bzw. Abschirmung sein.

In einer besonders bevorzugten Ausführungsform besteht die teildurchlässige Wand aus einem Silikatglas, welches für Wellenlängen unterhalb 200 nm undurchlässig ist. Derartige Gläser, häufig synthetische Quarzgläser, sind dem Fachmann bekannt.

Sofern ein röhrenförmiger UV-Strahler verwendet wird, kann eine zusätzliche Außenröhre aus der Abschirmung, z.B. ein Glasrohr aus dem genannten Silikatglas, über den Strahler gezogen werden. Ein solches Rohr dient gleichzeitig der Ausfilterung kurzer Wellenlängen und dem Schutz des Strahlers vor Verschmutzung, da das Schutz- und Abschirmungsrohr leichter ausgetauscht und gereinigt werden kann. Das zusätzliche Außenrohr führt jedoch gleichzeitig zu einem Lichtintensitätsverlust (Leistungsverlust). Als vorteilhafter wird es daher angesehen, wenn die Außenwand des Strahlers selbst aus einem Material besteht, welches den Wellenlängenausschluss unterhalb 200 nm bewirkt.

Ein stabförmiger Strahler kann auch mit Hilfe mehrerer kurzer Strahlerelemente oder zusammengesetzt aus Punktlichtquellen geeigneter Wellenlänge dargestellt werden.

Das Verfahren wird besonders vorteilhaft so geführt, dass der Luftstrom in einem Volumenstrom bis zu 250 m³/Stunde an dem wenigstens einen Strahler vorbeigeführt wird. Bei diesen Durchsätzen und üblichen Strahlerdurchmessern von ca. 15 bis 30 mm kann die Gerätegeometrie leicht vom Fachmann in einer für das erfindungsgemäße Verfahren geeigneten Weise angelegt werden.

Der Luftstrom wird in einer bevorzugten Ausführungsform zusätzlich einer Ionisation ausgesetzt. Vorzugsweise werden bipolare Ionisationsröhren verwendet. Die Ionisation kann vorteilhafter Weise der UV-Bestrahlung nachgeschaltet sein bzw. erfolgt stromabwärts von dieser. Sie dient in erster Linie dazu, die Luft zusätzlich mit "aktiviertem Sauerstoff" anzureichern. Hierfür können kommerziell erhältliche, mit Hochspannung arbeitende Ionisationsröhren eingesetzt werden. Erfahrungsgemäß wird mit Hilfe der Luftionisation eine besonders reine, frische und angenehme Raumluft erzeugt. Der aktivierte Sauerstoff neutralisiert Gerüche, falls vorhanden, und lädt die Luft auf. Die aktivierte Luft wird aus dem Gerät ausgetragen.

Die Ionisation kann vorzugsweise auch geregelt erfolgen und je nach Bedarf zu- und abgeschaltet werden. Für die Regelung wird in einem besonders bevorzugten Ausführungsbeispiel ein integrierter Luftqualitätssensor verwendet, der die flüchtigen organischen Bestandteile misst (VOC-Sensor, kommerziell erhältlich), mit einem zuvor für die jeweilige Entkeimungsaufgabe festgelegten Maximalwert vergleicht und die Intensität der Luftionisation über die Ausgangsspannung der Ionisationsröhren zwischen Null und dem Maximalwert regelt bzw. nachführt.

Ferner ist es bevorzugt, wenn die Luft vorab einen Partikelfilter durchläuft. Dieser dient nur der Ausfilterung von Rauch, Staub und gröberen Teilchen und sollte vorzugsweise kein HEPA-Filter sein, da sich ein solcher Feinstaubfilter zusetzen und dann als Nährboden für die eingangs noch vorhandenen Keime dienen kann. Als Staub- oder Partikelfilter ist ein Vlies, ein feinmaschiges Gitter, z.B. aus Metall, oder ein Kohlefilter geeignet. Bevorzugt wird eine Filtermatte verwendet, die von außen leicht austauschbar angeordnet wird.

Schließlich kann in einer weiteren Stufe eine katalytische Oxidation an einem Aktivkohle- oder Metalloxid-beschichteten Substrat (Katalysator) erfolgen. Der Katalysator wird - falls gewünscht - der UV-Bestrahlung und vorzugsweise auch der Ionisation nachgeschaltet. Er dient der Nachbehandlung in Bezug auf Gerüche und mit einer UV-Behandlung nicht zu erfassender Schadstoffe.

Die Aufgabe der Erfindung wird außerdem gelöst durch eine Vorrichtung zur Entkeimung strömender Luft mit UV-Strahlung, die insbesondere zur Durchführung des erfindungsgemäßen Verfahrens geeignet ist.

Die erfindungsgemäße Vorrichtung umfasst wenigstens einen Behandlungsraum und wenigstens einen in dem Behandlungsraum angeordneten stabförmigen UVC-Strahler sowie eine apparativ verwirklichte Zwangsführung in dem Behandlungsraum, mit deren Hilfe die strömende Luft quer zur Längsachse des stabförmigen UVC-Strahlers durch einen zwischen Strahleroberfläche und einem Vorrichtungsteil oder der Oberfläche eines weiteren Strahlers gebildeten Spalt hindurchgeführt wird, wobei der oder die Strahler über den gesamten Spaltquerschnitt eine Strahlungsleistung von mindestens 30 mW/cm² erzeugt/erzeugen.

Die Zwangsführung wird einerseits durch die Geometrie des Behandlungsraums, d.h. der Behandlungskammer selbst sowie durch zusätzliche Einbauten, wie beispielsweise Luftleitbleche verwirklicht. Der Spalt, durch den der Luftstrom der zu behandelnden Luft hindurchgeführt wird, besteht entweder längs des stabförmigen Strahlers zwischen dessen Oberfläche und einem Vorrichtungsteil, nämlich einer Wand des Behandlungsraumes oder einem strömungslenkenden Einbau wie einem Leitblech, oder der Spalt wird durch zwei benachbarte im wesentlichen parallel zueinander verlaufende Strahler gebildet. Innerhalb eines Behandlungsraumes können auch mehrere Spalte vorhanden sein, die dann jeweils den Bedingungen dieser Erfindung genügen.

Die gewünschte Strahlungsdichte kann mit verschiedenen kommerziell erhältlichen Strahlern erzielt werden. Eine Übersicht gibt Tabelle 1.

**Tabelle 1**

| Leistung UV-Strahler* (ozonfreier Quecksilber-Niederdruckstrahler) mW/cm² | | | | |
|---|---|---|---|---|
| Abstand zum Sensor (cm) | Lampenleistung | | | |
| | 6 W | 18 W | 36 W | 58 W |
| | | | | |
| 5 | 39,10 | 57,00 | 53,00 | 76,90 |
| 7 | 27,30 | 40,10 | 38,20 | 55,60 |
| 9 | 20,70 | 32,30 | 31,00 | 45,40 |
| 11 | 15,80 | 27,60 | 27,30 | 38,00 |
| 13 | 12,70 | 24,30 | 25,00 | 34,60 |

| | | | | |
|---|---|---|---|---|
| *Messgerät DR. Gröbel RM11, Gemessene Wellenlänge 254 nm | | | | |

Bevorzugt wird für die wenigstens eine UVC-Strahlungsquelle, d.h. den wenigstens einen UVC-Strahler eine Quecksilberlampe (z.B. Quecksilberdampflampe, Amalgamlampe) oder eine Excimerlampe verwendet. Besonders geeignet sind Quecksilber-Niederdruck- oder Quecksilber-Mitteldrucklampen, wie sie im Handel erhältlich sind. Die Werte in Tabelle 1 wurden mit einer Reihe ozonfreier Quecksilber-Niederdruckstrahler ermittelt.

In einer sehr bevorzugten Ausführungsform ist der Strahler rohrförmig und quer zur Strömungsrichtung des Luftstroms angeordnet. Der (jeweilige) Spalt entlang der Oberfläche der Strahlungsquelle oder der Strahlungsquellen besitzt bevorzugt die Form eines Längsschlitzes parallel zur Längsachse des stabförmigen Strahlers und wird vorzugsweise zwischen der Strahleroberfläche und zwangsführenden Einbauten oder Gehäuseverengungen realisiert.

Wie anhand des Verfahrens schon beschrieben, ist die UVC-Strahlungsquelle vorzugsweise wenigstens in Abstrahlungsrichtung von einem schützenden Glaskörper umgeben, der für Licht mit Wellenlängen unterhalb 200 nm nicht durchlässig ist. Hierbei kann es sich, wie oben näher beschrieben, um die Strahleraußenwand oder ein zusätzliches Schutzrohr handeln.

Die Vorrichtung beinhaltet Mittel zum Fördern der zu entkeimenden Luft durch den Behandlungsraum, wenn die Luft der Vorrichtung nicht ohnehin strömend zugeführt und durch sie hindurchgeführt wird, wie zum Beispiel innerhalb des Strömungskanals einer Klimaanlage. Als Modul innerhalb einer Klimaanlage benötigt die Vorrichtung keine eigenen Luftfördermittel. Als Mittel zum Fördern der Luft kann ein Ventilator oder Lüfter eingesetzt sein. Dieser wird - soweit erforderlich - bevorzugt stromabwärts der UVC-Strahler positioniert, saugt dann die Luft von außen durch den UV-Behandlungsraum und befördert sie zu den weiteren Behandlungsstationen und/oder nach außen.

Die Vorrichtung kann in einer möglichen Ausführungsform stromaufwärts vom UVC-Strahler, d.h. vorgeschaltet vor dem (ersten) Strahler, einen Filter enthalten. Dabei handelt es sich um einen Partikelfilter, der gröbere Partikel oberhalb ca. 10 µm Querschnitt (Maschenweite) oder weiter vorzugsweise oberhalb ca. 5 µm Querschnitt (Maschenweite) abhält.

Die Vorrichtung kann, wenn dies gewünscht wird, stromabwärts des UVC-Strahlers oder der UVC-Strahler wenigstens eine zusätzliche Luftbehandlungseinrichtung aufweisen. Hierbei kann es sich um Filtereinheiten, Katalysatoreinheiten, Luftbefeuchtungseinheiten und anderes mehr handeln.

Die Vorrichtung kann gemäß einer weiteren Ausführungsform eine Luftionisationseinheit enthalten. Die Luftionisation arbeitet in der Regel mit Hochspannungsionisationsröhren, die für den Zweck bekannt sind. Die Luftionisationseinheit bzw. die Ionisationsröhren befinden sich bevorzugt stromabwärts der UVC-Strahler und weiter bevorzugt unmittelbar vor der oder den Luftaustrittsöffnung(en). Die Ionisationseinheit kann geregelt sein, vorzugsweise anhand der Luftqualität in Bezug auf zuvor festgelegte Kriterien. Auf Basis der Vorgabewerte wird die Leistung der Luftionisation erhöht oder erniedrigt. Als Sensoren zur Ermittlung der Stellgröße eignen sich vorzugsweise VOC-Sensoren, die den Gehalt an flüchtigen organischen Bestandteilen in der Luft bestimmen.

Ferner kann die Vorrichtung gemäß einer weiteren Ausführungsform eine Katalysatoreinheit für eine katalytische Oxidation umfassen. Hierbei werden vor allem Schad- und Geruchsstoffe in der Luft mit Hilfe des Luftsauerstoffs an dem Katalysator oxidiert und so oxidativ zerlegt und unschädlich gemacht. Als Katalysator ist beispielsweise ein Aktivkohlekatalysator ein keramischer Katalysator oder ein Metalloxidkatalysator geeignet, wie er für entsprechende Zwecke bekannt ist. Die Katalysatoreinheit befindet sich - soweit vorhanden - bevorzugt stromabwärts, also in Strömungsrichtung hinter den UV-Strahlern und - falls solche vorhanden sind - weiter vorzugsweise hinter den Ionisationsröhren.

Im Folgenden wird die Erfindung anhand von in den Figuren gezeigten Ausführungsbeispielen näher erläutert. In der Zeichnung zeigen:
- Figuren 1 und 2: ein erstes Luftentkeimungsgerät, Figur 1 Längsschnitt, Figur 2 Querschnitt;
- Figur 3: eine weitere Ausführungsform für ein Luftentkeimungsgerät, als Standgerät ausgeführt in einem Teillängsschnitt;
- Figur 4: das Gerät aus Figur 3 in einem vollständigen Längsschnitt;
- Figur 5: eine Bestrahlungseinheit als Modul für eine Klimaanlage im Längsschnitt;
- Figur 6: den UV-Behandlungsbereich eines anderen Standgeräts im Teil-Längsschnitt.

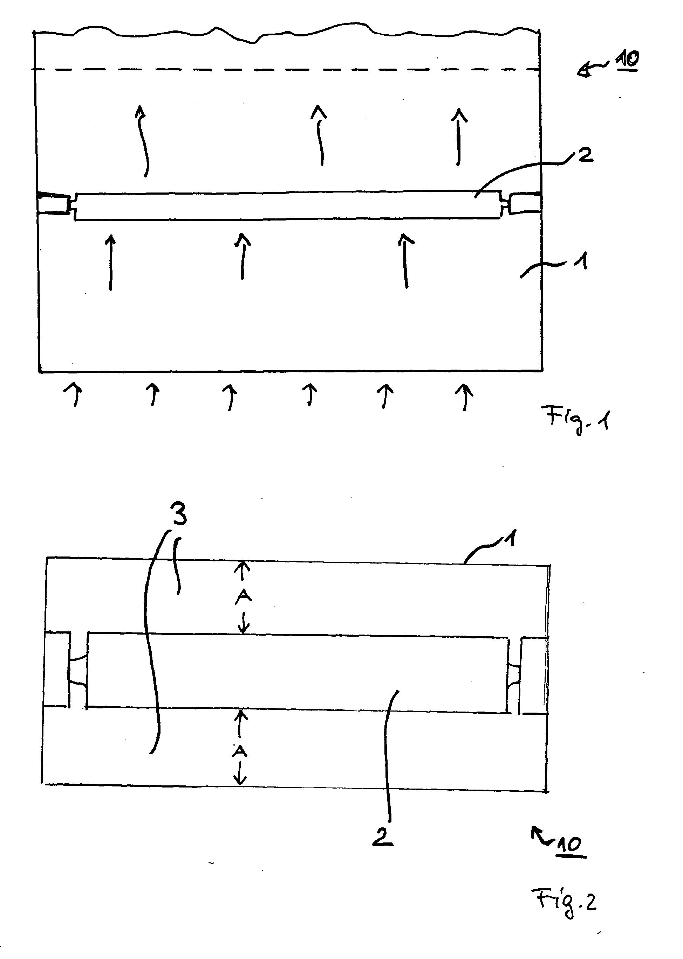

Figur 1 zeigt ein im Ganzen mit 10 bezeichnetes Luftentkeimungs-Wandgerät mit Lufteinlass von unten. Die zu behandelnde Luft strömt in Pfeilrichtung von unten nach oben parallel zur Wand durch das Gerät. Diese Strömungsrichtung wird normalerweise in Räumen durch die Konvektion aufsteigender wärmerer Luft vorgegeben, wenn das Gerät an einer Innenwand angeordnet ist. Mittel zum Fördern der Luft durch das Gerät können vorgesehen sein, sind jedoch in dieser Abbildung nicht dargestellt. Die von unten in den Behandlungsraum des Gerätes 10 bzw. der Vorrichtung einströmende Luft - hier durch Pfeile dargestellt - umströmt den Strahler 2 quer zu dessen Längsachse und verlässt das Gerät anschließend nach oben unmittelbar nach außen oder durch eine sich anschließende weitere Behandlungseinheit.

Figur 2 zeigt dasselbe Gerät in einer Querschnittsansicht. Durch die Oberfläche des Strahlers 2 und die Wände des Behandlungsraumes 1 werden hier zwei Spalte 3, jeweils mit dem Spaltquerschnitt A gebildet. Die maximale Spaltbreite A beträgt hier 30 mm von der Oberfläche des Strahlers 2 bis zur Geräteinnenwand. Als Strahler wird ein UVC-Hg-Niederdruckstrahler mit 6 W Nennleistung verwendet. Die Strahlungsleistung beträgt bei diesem Abstand ca. 54 mW/cm², gemessen bei 254 nm. Die Luftfördermenge wird bei einem mit Ventilator betriebenen Gerät zwischen minimal 13 m³/Stunde entsprechend 3,6 l/s und maximal 16 m³/Stunde entsprechend 4,4 l/s eingestellt.

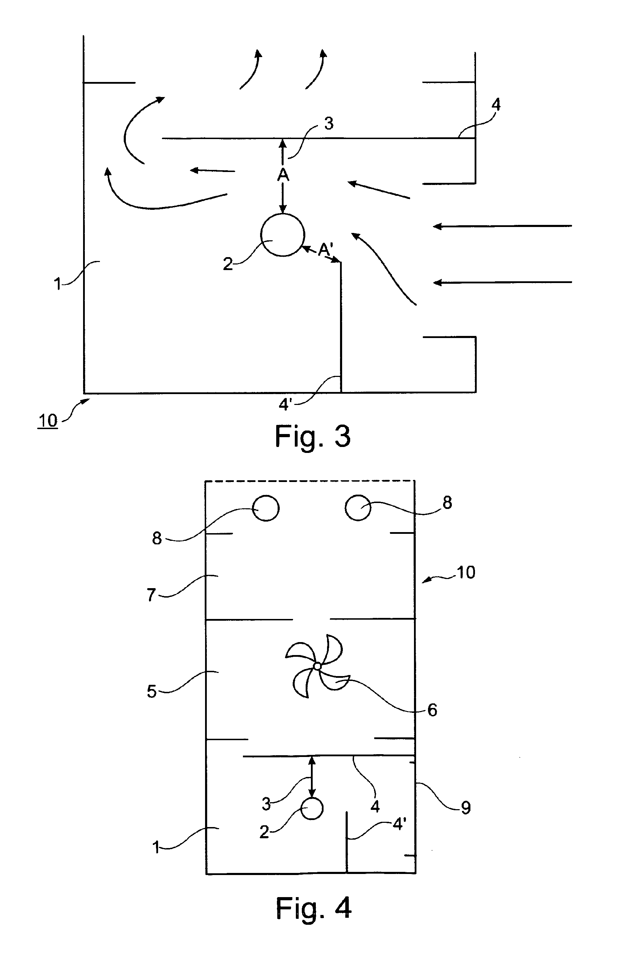

Figur 3 zeigt ein im Ganzen mit 10 bezeichnetes Luftentkeimungs-Standgerät in einem Teillängsschnitt, der den Behandlungsraum 1, d.h. die UV-Kammer, im Detail zeigt. Die in den UV-Behandlungsraum 1 eintretende Luft durchströmt zunächst einen in dieser Ansicht nicht dargestellten Staubfilter und wird sodann durch die Luftleitbleche 4 durch den Spalt 3 mit der Spaltbreite A gezwungen. In der Luft etwa vorhandene Keime können der intensiven Bestrahlung zwischen Strahler 2 und Leitblech 4 nicht ausweichen. Die Spaltbreite A' ist geringer als die Spaltbreite A. Durch die geringere Spaltbreite A' und die Strömungsführung werden zusätzliche Verwirbelungen verursacht, die alle in der Luft vorhandenen Teilchen relativ dicht an der Oberfläche des Strahlers 2 vorbeizwingen.

Bei dem Strahler 2 handelt es sich hier um einen 18 W-Strahler mit einem Lampenglaskörper aus synthetischem Quarzglas. Die UV-Intensität, gemessen bei 254 nm, beträgt hier ca. 100 mW/cm². Die Luftfördermenge wird bei diesem Standgerät auf Werte zwischen minimal 70 m³/Stunde entsprechend 19,4 l/s und maximal 230 m³/Stunde entsprechend 63,9 l/s eingestellt. Je nach Leistungsanforderung können in entsprechenden Standgeräten Strahler bis ca. 60 W eingesetzt werden. Die hohe Intensität führt aufgrund des Ausschlusses kurzer Wellenlängen nicht zur Ozonbildung. Es findet daher keine Belastung der Umgebungsluft mit Ozon statt.

Figur 4 zeigt das Standgerät aus Figur 3 in einem schematischen Gesamtlängsschnitt. Neben der Behandlungskammer 1 gibt es einen Lüfterraum 5, in dem wenigstens ein Lüfter 6 untergebracht ist. Dieser saugt die zu entkeimende Luft aus dem UV-Behandlungsraum 1 an und leitet sie in den Endbehandlungsraum 7 weiter. Kurz vor dem Ausgang des Geräts 10 sind lonisationsröhren 8 angeordnet, die die behandelte Luft mit aktiviertem Sauerstoff anreichern, der mit der behandelten Luft aus dem Gerät 10 in die Umgebung ausströmt. Nach der Behandlung ist die Luft vollständig entkeimt. Wie Tests zur Wirkung ergeben haben, sind pathogene Keime nach der erfindungsgemäßen Behandlung nicht mehr nachweisbar. Um grobe Schmutzpartikel und Rauch aus dem Gerät fernzuhalten, wird die Luft durch einen Eingangsfilter 9 angesaugt. Der Eingangsfilter 9 verhindert außerdem, dass UV-Strahlung ungewollt durch die Gehäuseöffnung nach außen tritt. Das Luftfassungsvermögen des in diesem Beispiel gezeigten Luftentkeimungsgerät beträgt ca. 180 m³, der Volumenstrom maximal 230 m³/Stunde.

Das Gerät 10 besitzt zusätzlich hier nicht dargestellte elektrische Komponenten, wie elektrische Anschlüsse, Schalter und Regler sowie zweckmäßiger Weise eine Zeiterfassung. Die Ionisationsröhren können in ihrer Leistung geregelt sein. In diesem Falle würde ein VOC-Sensor innerhalb des lonisationsraumes 7 unmittelbar vor dem Luftaustritt angeordnet.

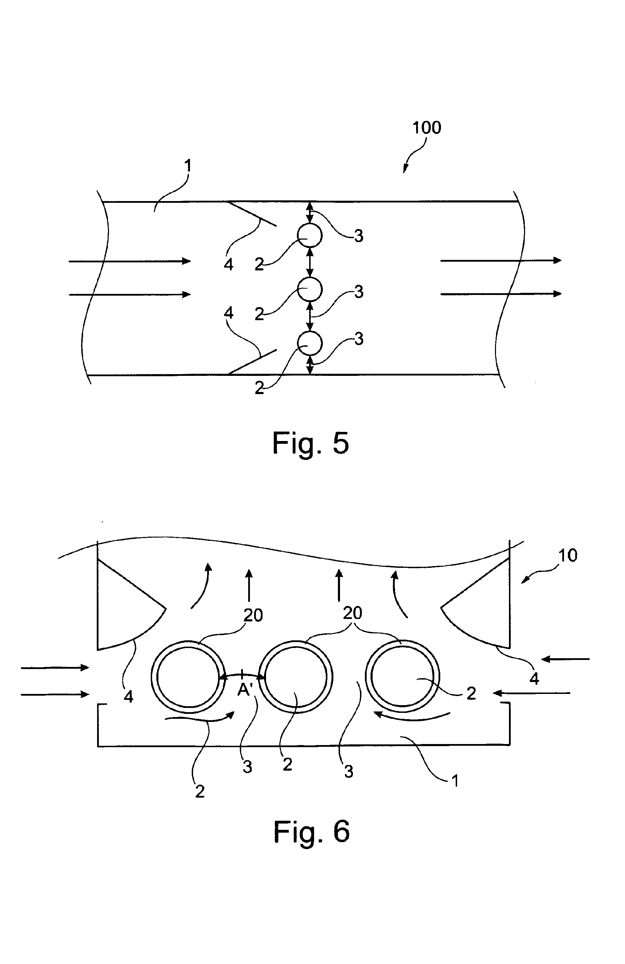

Figur 5 zeigt ein weiteres Ausführungsbeispiel, das als Modul für einen Luftkanal einer Klimaanlage vorgesehen ist. Das Modul, das durch die UV-Bestrahlungseinheit 100 gebildet wird, bildet einen Behandlungsraum 1 in Form eines Kanals mit quadratischem oder rechteckigem Querschnitt. Die Luft durchströmt den Behandlungsraum 1 von links nach rechts. Die strömende Luft kann durch einen hier nicht dargestellten Filter in das Modul 100 eintreten und passiert parallel in einem Rahmen angeordnete UVC-Strahler 2. Die Leitbleche 4 verbessern die Durchmischung der strömenden Luft und leiten den Luftstrom gezielter den Strahlern 2 zu. Die Spalte 3 werden hier nicht nur zwischen Gehäuse und Strahler 2, sondern auch zwischen zwei Strahlern 2 ausgebildet.

Figur 6 zeigt eine Teilschnittansicht für noch ein weiteres Ausführungsbeispiel. Der Behandlungsraum 1 im Bodenbereich eines Standgerätes ist mit drei UVC-Strahlern 2, die parallel zueinander so angeordnet sind ausgerüstet, so dass die Luftspalte 3 für den Durchtritt des Luftstroms zwischen dem Strahlern 2 oder in Verbindung mit den Leitblechen 4 gebildet werden. Im Übrigen zeigen entsprechende Bezugszeichen gleiche Bauteile.

### Wirksamkeitsuntersuchungen gegenüber dem H1N1-Influenzavirus (A/Hamburg/05/09)

Das Gerät wurde in einer Studie auf seine virusinaktivierenden Eigenschaften gegenüber dem Influenzavirus H1N1 (Stamm A/Hamburg/05/09) untersucht. Die Wirksamkeit der UV-Quelle des Geräts wurde evaluiert. Das Virus wurde in Kulturmedium mit 0,1 % bovinem Serumalbumin (BSA) für 60 Sekunden, 1 Sekunde und 0,5 Sekunden der UV-Strahlung ausgesetzt.

| Prüfbedingungen | |
|---|---|
| Prüftemperatur | 20,0 +/-0,5 °C |
| Strahlerabstand von Prüfsubstrat | 40mm |
| Strahler | Quecksilber-Niederdruckstrahler, 6 Watt, Nennleistung (Phillips®) |
| Strahlungsleistung in Prüfabstand | ca. 54 mW/cm² |
| Einwirkzeiten der UV-Strahlung in bauseitig bedingter Entfernung | 60, 1, 0,5 Sekunden |
| Eiweißbelastung | 0,2 % BSA |
| Verdünnungsmittel | Minimum Essential Medium (MEM) (Firma: GIBCO) |
| Virusstamm | Influenzavirus H1N1 (A/Hamburg/05/09) |

### Herstellung der Virussuspension

Zur Herstellung der Virussuspension wurden MDCK-Zellen eingesetzt, die in 175 cm² Roux-Flaschen (Nunc GmbH & Co, Wiesbaden) mit Minimum Essential Medium (MEM), unter Zusatz von 10 % Fetalem Kälberserum (FKS), Natriumpyrovat, Penicillin, Streptomycin und Glutamin gehalten worden sind. Kultivierte und zu 80 Prozent konfluente MDCK-Zellen wurde in 175 cm² Zellkulturflaschen mit dem Influenzavirus H1N1 (A/Hamburg/05/09) und einer multiplicity of infection (moi) von 0,1 infiziert. Die Zellen wurden mit 10 ml einer entsprechenden Virusverdünnung inokuliert und für eine Stunde bei 37 °C (5 % CO₂) im Brutschrank inkubiert. Im Anschluss wurden 30 ml MEM unter Zusatz von Glutamin, Penicillin, Streptomycin, 1 µg/ml TPCK-Trypsin und BSA mit einer Endkonzentration von 0,2 % und bis zum Auftreten eines zytopathischen Effekts bei 37 °C (5 % CO₂) im Brutschrank aufbewahrt. 72 Stunden nach Infektion wurden die Überstände geerntet. Der virushaltige Überstand wurde durch eine zehnminütige Zentrifugation bei 3000 Umdrehungen pro Minute (UpM) in einer Megafuge 1.0 R (Heraeus) von Zelltrümmern befreit und bei 4 °C aufbewahrt.

### Durchführung des Inaktivierungsversuchs

Die Suspension des Prüfvirus (80 µ) wurde auf einen Objektträger aufgebracht und für 60, 1 oder 0,5 Sekunden in bauseitig bedingten Abstand zur UV-Quelle gebracht. Die Einhaltung der kurzen Inkubationszeiten (1 und 0,5 Sekunden) wurden durch den Auslösemechanismus einer Belichtungseinheit gewährleistet. Die Viruskontrolle wurde analog der Produktprüflösung behandelt. Das Prüfverfahren fand bei 20,0 +/- 2 °C statt. Im Anschluss an die Bestrahlung wurde sofort eine Titration der Restinfektion in 10er-Schritten mit MEM mit Penicillin, Streptomycin, Glutamin, BSA und Trypsin angesetzt und die Verdünnungsreihe auf eine semikonfluent gewachsene Zellkultur in 96-Loch Platten durchgeführt. Die Inkubation der Zellkultur erfolgte bei 37 °C im CO₂-Brutschrank mit 5 % CO₂-Gehalt.

### Bestimmung der Infektiosität

Die Infektiosität wurde im Quantalversuch (Endpunkttitration) bestimmt. Für diesen Versuch wurden 96-Loch Platten mit konfluierenden MDCK-Zellagen verwendet. Je vier Vertiefungen wurden mit 100 µl der Verdünnungsreihen beimpft. Danach wurden die Zellkulturplatten für 3 Tage bei 37 °C im CO₂-Brutschrank mit 5 % CO₂-Gehalt in einer feuchten Kammer inkubiert. Die zytopathischen Effekte wurden anhand der Auswertung am Lichtmikroskop dokumentiert. Anschließend wurde die Virusverdünnung ermittelt, bei der 50 % der beimpften Kulturen eines Verdünnungssatzes infiziert waren (TCID₅₀/ml). Die Berechnung der TCID₅₀/ml erfolgte nach dem Spearman-Kärber-Verfahren (Br. J. Psychol. 2 (1908): 227-42, Arch. exp. Path. Pharmak. 162 (1931): 480-87).

### Berechnung der virusinaktivierenden Wirksamkeit

Die Beurteilung der virusinaktivierenden Wirkung erfolgte durch die Berechnung des Titerabfalls (Δ lg TCID₅₀) gegenüber der jeweils parallel mitgeführten Kontrolltitration.

### Ergebnis

### Testung der Reduktion der Infektiösität nach UV-Bestrahlung

(a) Ohne UV-Bestrahlung - 7,5 TCID₅₀/ml
(b) 60 Sekunden UV-Bestrahlung - keine Infektiösität
(c) 1 Sekunden UV-Bestrahlung - keine Infektiösität
(d) 0,5 Sekunden UV-Bestrahlung - keine Infektiösität

**Tabelle 2**

| | | | | | | | | |
|---|---|---|---|---|---|---|---|---|
| | 10⁻¹ | 10⁻² | 10⁻³ | 10⁻⁴ | 10⁻⁵ | 10⁻⁶ | 10⁻⁷ | 10⁻⁸ |
| (a) | X | X | X | X | X | | | |
| H1N1 | X | X | X | X | X | | | |
| ohne | X | X | X | X | X | | | |
| UV | X | X | X | X | X | | | |
| | 10⁻¹ | 10⁻² | 10⁻³ | 10⁻⁴ | 10⁻⁵ | 10⁻⁶ | 10⁻⁷ | 10⁻⁸ |
| (b) | | | | | | | | |
| H1N1 | | | | | | | | |
| 60 Sek. | | | | | | | | |
| UV | | | | | | | | |
| | 10⁻¹ | 10⁻² | 10⁻³ | 10⁻⁴ | 10⁻⁵ | 10⁻⁶ | 10⁻⁷ | 10⁻⁸ |
| | 10⁻¹ | 10⁻² | 10⁻³ | 10⁻⁴ | 10⁻⁵ | 10⁻⁶ | 10⁻⁷ | 10⁻⁸ |
| (C) | | | | | | | | |
| H1N1 | | | | | | | | |
| 1 Sek. | | | | | | | | |
| UV | | | | | | | | |
| | 10⁻¹ | 10⁻² | 10⁻³ | 10⁻⁴ | 10⁻⁵ | 10⁻⁶ | 10⁻⁷ | 10⁻⁸ |
| | 10⁻¹ | 10⁻² | 10⁻³ | 10⁻⁴ | 10⁻⁵ | 10⁻⁶ | 10⁻⁷ | 10⁻⁸ |
| (d) | | | | | | | | |
| H1N1 | | | | | | | | |
| 1 Sek. | | | | | | | | |
| UV | | | | | | | | |

Zusammenfassend kann gesagt werden, dass die Behandlung mit dem Gerät inaktivierende Eigenschaften gegen H1N1-Influenzaviren zeigt, deren Infektiösität durch die Behandlung unter die Nachweisgrenze sinkt.

## Patentansprüche

1. Verfahren zur Entkeimung potentiell mit pathogenen Keimen beladener Luft, bei welchem die Luft innerhalb eines Gerätes (10) oder einer Bestrahlungseinheit (100) in einem Luftstrom geführt und mit UVC-Licht bestrahlt wird, **dadurch gekennzeichnet, dass** der Luftstrom quer zur Längsachse wenigstens eines stabförmigen UVC-Strahlers (2) durch wenigstens einen, angrenzend an die Oberfläche des Strahlers (2) ausgebildeten Spalt (3) hindurchgeführt wird, wobei der Luftstrom so zwangsgeführt wird, dass jedes Teilvolumen während des Durchtritts einer Strahlungsleistung von mindestens 30 mW/cm2 ausgesetzt wird.

2. Verfahren nach Anspruch 1, **dadurch gekennzeichnet, dass** die UVC-Bestrahlung praktisch ausschließlich mit Licht oberhalb 200 nm Wellenlänge erfolgt.

3. Verfahren nach Anspruch 1 oder 2, **dadurch gekennzeichnet, dass** der Luftstrom in einem Volumenstrom bis zu 250 m3/h an dem wenigstens einen Strahler (2) vorbeigeführt wird.

4. Verfahren nach einem der Ansprüche 1 bis 3, **dadurch gekennzeichnet, dass** der Luftstrom zusätzlich einer Ionisation ausgesetzt wird.

5. Verfahren nach einem der Ansprüche 1 bis 4, **dadurch gekennzeichnet, dass** die Luft vorab einen Partikelfilter (9) durchläuft.

6. Vorrichtung (10) zur Entkeimung strömender Luft mit UV-Strahlung, insbesondere zur Durchführung des Verfahrens nach einem der Ansprüche 1 bis 5, mit einem Behandlungsraum (1) und wenigstens einem in dem Behandlungsraum (1) angeordneten stabförmigen UVC-Strahler (2) **gekennzeichnet durch** eine Zwangsführung in dem Behandlungsraum (1), mit deren Hilfe die strömende Luft quer zur Längsachse des stabförmigen UVC-Strahlers (2) **durch** einen zwischen Strahleroberfläche und einem Vorrichtungsteil (4, 4') oder der Oberfläche eines weiteren Strahlers (2) gebildeten Spalt (3) hindurchgeführt wird, wobei der oder die Strahler (2) über den gesamten Spaltquerschnitt (A; A') eine Strahlungsleistung von mindestens 30 mW/cm2 erzeugt/erzeugen.

7. Vorrichtung nach Anspruch 6, **dadurch gekennzeichnet, dass** als UVC-Strahler (2) ein Quecksilberstrahler oder ein Excimerstrahler verwendet wird.

8. Vorrichtung nach Anspruch 6 oder 7, **dadurch gekennzeichnet, dass** der Spalt (3) parallel zur Längsachse des stabförmigen Strahlers (2) zwischen der Strahleroberfläche und zwangsführenden Einbauten (4; 4') oder Gehäuseverengungen ausgebildet ist.

9. Vorrichtung nach einem der Ansprüche 6 bis 8, **dadurch gekennzeichnet, dass** der oder die UVC-Strahler (2) wenigstens in Abstrahlungsrichtung durch einen Glaskörper (20) abgeschirmt ist, der für Licht mit Wellenlängen unterhalb 200 nm nicht durchlässig ist.

10. Vorrichtung nach einem der Ansprüche 6 bis 9, **dadurch gekennzeichnet, dass** sie Mittel (6) zum Fördern der zu entkeimenden Luft durch den Behandlungsraum beinhaltet.

11. Vorrichtung nach einem der Ansprüche 6 bis 10, **dadurch gekennzeichnet**, das sie stromabwärts des UVC-Strahlers (2) wenigstens eine zusätzliche Luftbehandlungseinrichtung aufweist.

12. Vorrichtung nach Anspruch 11, **dadurch gekennzeichnet, dass** sie eine vorzugsweise in Abhängigkeit von der Luftqualität geregelte Luftionisationseinheit (7; 8) enthält.
